# EUROPEAN PATENT APPLICATION

(11) **EP 2 835 130 A1**
(43) Date of publication of application: **11.02.2015**
(21) Application number: 13179245.9
(22) Date of filing: 05.08.2013
(51) Int. Cl.: A61K 8/34, A61K 8/73, A61Q 19/08, A61K 8/97

(54) **Composition for skin anti-ageing treatment**

(71) Applicant: Polichem SA, 1526 Luxembourg (LU)
(72) Inventor: Scarci, Francesco, I-22100 Como (IT); Iob, Giuliana, CH-6953 Lugaggia (CH); Mailland, Federico, CH-6900 Lugano (CH)
(74) Representative: Pistolesi, Roberto

(57) **Abstract**

The present invention is directed to a composition comprising an extract of *Humulus* lupulus combined with hyaluronic acid and a C₁-C₄ alkanol, and to its use as an anti-ageing treatment for the skin of human being. For the use of the present invention, the new composition is administered topically.

## Description

The present invention is directed to a composition comprising an extract of *Humulus lupulus* combined with hyaluronic acid and a C₁-C₄ alkanol, and to its use as an anti-ageing treatment for the skin of human being. For the use of the present invention, the new composition is administered topically.

### BACKGROUND OF THE INVENTION

The ageing process is the physiological result of the cellular senescence and the related declining ability to respond to stress. This irreversible process leads to some changes in the body: one of them is related to the appearance of facial wrinkles.

Skin is made of two main layers: the outer one is the epidermis, made mainly by keratinocytes, responsible for the formation of a barrier against environmental damages (pathogens, heat, UV radiation and water loss). The inner layer, the dermis, contains the connective tissue, made by structural components such as collagen (responsible for the skin firmness), elastic fibres (responsible of the skin elasticity) and extracellular matrix (structural component). There is also a third layer of subcutaneous tissues, containing fat cells that provide insulation to the body.

The wrinkle is a fold, ridge or crease in the skin, due to a process of glycation that impairs the functioning of biomolecules (Danby FW, "Nutrition and aging skin: sugar and glycation", Clin Dermatol 2010,28(4):409-411). The ageing process is not the only cause of wrinkles appearance, other factors can promote the wrinkling: they are smoking, sun exposure, skin type, environmental factors and genetic heredity (Demierre MF et al, "Public knowledge, awareness, and perceptions of the association between skin aging and smoking", J Am Acad Dermatol, 1999,41(1) :27-30).

The formation of skin wrinkles consists in a failure of the skin structures due to a lack of collagen or to its modification, thinning and/or fractioning, due to the stretching and repeated extension of some areas of the skin, especially the face (Fisher GJ, "The Pathophysiology of Photoaging of the Skin" Cutis, 2005,75(2S):5-9). Also the lack of elastin has an important role in the process. Its decrease and its consequently loss of elasticity, causes the increase of volume of the skin and phenomena such as the double chin. All these combined changes in the scaffolding of the skin, cause the appearance of the wrinkles, and the nature of the wrinkles depends on the nature of skin and muscle contraction. The consequences of this degenerative process lead to enhanced skin fragility, a decrease of the amount of nutrients available to the epidermis, interfering with the normal skin repair process, therefore bringing to more noticeable wrinkling and sagging process.

Habitual facial expressions and therefore muscles permanently contract, cause the skin to wrinkle due to loss of tissues as above. Moreover, the effects of gravity are responsible for the appearance of wrinkles and skin sagging. This provokes jowls and drooping eyelids.

Thus, ageing is an irreversible process affecting the skin, due to a decrease of structural substances included in the layers or in imperfect remodelling of the fibres (mainly collagen) and other multifactorial issues that cause the formation of wrinkles.

Regeneration of the lost tissues, including mainly collagen fibres and elastin, should be the best target for wrinkle treatment and prevention.

In the art, there are a lot of treatments useful for ameliorating the wrinkles, with medical, surgical and cosmetic solutions. These treatments are intended to change the nature of aging collagen, stretch the skin, fill in the depressions in the skin, or paralyze muscles that cause the skin to crease.

Among the medical treatments there are: Vitamin A Acid (retinoids), which acts increasing the turnover of skin cells, but they may produce redness, peeling and general discomfort; alpha-hydroxy acids penetrate into the top of the layer skin, producing only subtle improvement though and causing a mild and temporary irritation; the anti-aging Serum stimulates the skin to rebuild the collagen and elastin network in the dermis resulting in a renewal of skin structure, improved skin elasticity and smoothing of wrinkles. The disadvantages however, are related the cost and the fact that very often the real composition of this compounds, obtained from an animal source, is not specified.

In the surgical field, there are a lot of techniques: dermabrasion and laser resurfacing that act sanding the skin down, by means of a rotating instrument and laser, respectively. Even if all these treatments could get an excellent improvement, they can also produce significant side effects, including scarring and permanent changes in skin color.

Plastic surgical procedures (surgical facelift), injections of botulin toxin that relax the facial muscles and let the lines disappear, fillers (made on collagen or hyaluronic acid and calcium hydroxyapatite, or autologous fat transfer) that increase volume and flatten wrinkles and fold; heat and radiofrequency are other techniques that provide good improvements in the treatment of ageing. These improvements last some months then they must be repeated to sustain improvement. Again, surgical procedures are opposite to the tissue regeneration process, acting only in the aesthetic way and not in the sense of skin rejuvenation.

Among the cosmetic products there are: superficial or deeper peels that act in smoothing fine lines and scarring; microdermabrasion that acts as mild exfoliating agent. Both act opposite to the tissue regeneration, mostly by trying to improve the skin depressions by keeping out the uppermost skin layer.

Antioxidants provide a sun protection, neutralizing the free radicals responsible for the collagen breakdown: by this mechanism, antioxidants may be very important in the prevention of the further worsening of wrinkle forming. Finally, moisturizers can make wrinkles look temporarily less prominent, keeping the skin hydrated.

As a conclusion, the best product to treat and prevent wrinkle formation should act in the sense to: 1) regenerating tissues, mostly collagen, 2) opposing to oxidation, 3) moisturizing the skin.

Clinical and visual evaluations (wrinkles grade at level of nasolabial folds and crow's feet, malar region ptosis, surface microrelief, skin dullness and skin firmness), as well as instrumental evaluations, represent the methods needed for the determination of the efficacy of an anti-ageing treatment (Grove GL et al, Optical Profilometry: an objective method for quantification of facial wrinkles J Am Acad Dermatol, 21:631-637,1989). Wrinkles at level of nasolabial folds and on the area around the eyes are determined by means of two reference clinical and photographic scales, that identify or not, the presence and the grade of wrinkles, scoring the result from 0 (no wrinkles) to 7 (very marked wrinkles). Similarly, submental ptosis is scored from 0 (absence of ptosis - very regular oval face) to 5 (very marked ptosis - very irregular oval face) according to a clinical photographic scale (Monheit GD et al. Development and validation of a 6-point grading scale in patients undergoing correction of nasolabial folds with collagen implant. Dermatol Surg 2010;36:1809-1816). Based on a photographic scale cheek, also the surface microrelief is evaluated according to the referring score from 1 (very regular) to 4 (very irregular).

Skin dullness of the overall face evaluates the luminosity according to the score from 1 (luminous skin) to 4 (very opaque skin); as well as the skin firmness, that assesses the skin resistance to pinching, resistance to traction and recovery after pinching at level of cheek (malar region) according to the score from 0 (very important) to 4 (very weak).

In the art, *Humulus lupulus* (commonly called hops) is already known as an anti-wrinkle agent, though at a very high content, as we will see thereafter. It is a species of plant in the Cannabaceae family, main ingredient of many beers and other brewed beverages. Hop strobile contains resinous bitter principles (5 - 30%), mostly alfa-bitter acids (humulones 2 - 10%) and beta-bitter acids (lupulones 2 - 16%) and their oxidative degradation products (2-methyl-3-buten-2-ol); polyphenolic condensed tannins (2-4%); volatile oil (0.35 - 1.0%), mainly monoterpenes and sesquiterpenes (beta-caryophyllene, farnesene, humulene, beta-myrcene); chalcones (xanthohumol); flavonoids (kaempferol, quercetin, rutin); phenolic acids; and aminoacids (Bradley, 1992;Bruneton,1995; ESCOP,1997; Leung and Foster,1996; Newall et al., 1996;Wichtl and Bisset, 1994. For pharmaceutical purposes, plant extracts are preparations of liquid (e.g. liquid extract and tinctures), semi-solid (soft extracts and oleoresins) or solid (dry extracts) consistency, defined by their production process (state of the herbal drug to be extracted, solvent, extraction conditions) and their specifications. Extracts are prepared by suitable methods (e.g. maceration, percolation), using water, ethanol or other suitable organic solvent that comply with any relevant monograph of the Pharmacopoeia. The herbal drug to be extracted may undergo a preliminary treatment, for example, inactivation of enzymes, grinding or defatting. (Ph.Eur.7.0 04/2008:0765).

Hops have a proven history of herbal use, where they are employed mainly for their already known soothing, sedative, tonic and calming effects on the body and the mind (Schiller et al, Sedating effects of Humulus lupulus L. extracts, Phytomedicine. 2006;13(8):535-41).

WO2007/085327A1 discloses the vaginal use of *Humulus lupulus* extracts to prevent vaginal dryness in postmenopausal women at concentrations of *H. lupulus* as low as to avoid any pro-estrogenic effect. Use of *Humulus lupulus* to relieve signs of skin ageing and to lead wrinkles be less evident or disappear has been disclosed in a paper where xanthohumol, one of the flavonoids isolated from hop plant, at two different concentrations (0.1% and 1%), resulted efficacious in improving skin structure and firmness (Philips N et al, "Direct inhibition of elastase and matrixmetalloproteinases and stimulation of biosynthesis of fibrillar collagens, elastin, and fibrillins by xanthohumol", J Cosmet Sci, 2010,61(2):125-32). Xanthohumol represents 1% of the total content of *Humulus lupulus* drug (Milligan SR et al, "The endocrine activities of 8-prenylnaringenin and related hop (Humulus lupulus L.) flavonoids" J Clin Endocr Metab, 2000,85(12)4912-5) and the two concentrations effective according to Philips are very high and they cannot be reached in an anti-wrinkle cream or the like, simply by using a mother tincture or an extract. In order to reach the concentrations active on wrinkles in the cited art, sophisticated and expensive extractive methods have to be employed.

Another ingredient used against wrinkles is Hyaluronic acid. It is a natural constituent of the human body that can be found in the epithelial and conjunctive tissues. It provides three main functions: the protection of cartilages between the joints from mechanical deterioration, keeping them hydrated and controlling the cell migration. It has also a fundamental role in the stimulation of the immune responses by helping the white cells to fight several types of infections. Due to its effective biological hydrating nature and its regenerative properties, hyaluronic acid is used as a main ingredient in many anti-aging face creams and serums, though to be really effective, it is needed in concentrations equal or higher than 0,1% (Pavicic T, Efficacy of cream-based novel formulations of hyaluronic acid of different molecular weights in anti-wrinkle treatment, J Drugs Dermatol 2011,10:990-1000).

Ethanol is the most common organic solvent, widely used both at home and in industry. Similar effects are obtained by using ethanol or other low-molecular weight alcohols, i.e. lower alkanols, like propyl alcohol, isopropyl alcohol and the like. Ethanol and other lower alkanols are widely used in products with direct exposure to the human skin, like medical wipes and in most common antibacterial hand sanitizer gels due to its capability to kill organisms by denaturing their proteins and dissolving their lipids, being effective against many bacteria and fungi. On the contrary, ethanol, as well as propyl or isopropyl alcohols, are not recommended in compositions for skin rejuvenation/wrinkles, because their lipid dissolving effect and dehydrating effect may worsen the skin ageing and wrinkle appearance, by thinning and hardening the skin.

### DESCRIPTION OF THE INVENTION

It has now been surprisingly found that a C₁-C₄ alkanol, such as ethanol, can act synergistically with *Humulus lupulus* and hyaluronic acid, and is of benefit in the achievement of anti-ageing effect, in terms of collagen regeneration, antioxidant effect and moisturisation when applied on ageing skin.

A further advantage of this synergistic combination is that it is active at very low concentrations of the main ingredients, e.g. up to 15% by weight of *Humulus lupulus* extract, containing therefore a much lower dose of xanthohumol than disclosed by Philips N et al, avoiding any estrogenic effect. Similarly, the combination is active at very low concentrations of hyaluronic acid, up to 5% by weight, saving the high cost of this component.

The object of the present invention is a composition comprising a C₁-C₄ alkanol in combination with an extract of Humulus *lupulus* and hyaluronic acid, and its use as an anti-ageing treatment for the skin of human beings.

For the anti-ageing treatment as for the present invention, the combination of low concentrations of *Humulus lupulus* with low concentration hyaluronic acid and a C₁-C₄ alkanol, preferably ethanol, is administered topically in the form of semi-solid or liquid formulations; such formulations may be in the form of solutions, emulsions or suspensions, creams, gels, serum and ointments. They are particularly suitable to achieve an anti-ageing effect by direct application over the facial surface.

Such extract of *Humulus lupulus* may be a liquid, semi-solid or solid extract, preferably a liquid extract and more preferably a tincture, made with fresh plant strobiles, the so called "mother tincture" (Ph.Eur.7.3, 01/2012:2029). Such extract may be obtained by macerating the fresh female strobiles of *Humulus lupulus* for 20 days to 30 days into a solution of water and ethanol, at room temperature (preferably from 20 to 25 °C). Water is normally used in a weight ratio of 35.0% to 55.0% with respect to ethanol.

The extract of *Humulus lupulus,* may be present in w/w concentrations of from 0.1% to 15%, more preferably from 0.2% to 5%, most preferably from 0.5% to 2.5%.

Hyaluronic acid may be used as such or in the form of a pharmaceutically acceptable salt or ester. Pharmaceutically acceptable salts may be selected from sodium salt, potassium salt, calcium salt or a salt with a natural aminoacid (e.g. lysine, arginine, methionine or aspartic acid). Pharmaceutically acceptable esters may be selected from ascorbyl hyaluronate, palmitoil hyaluronate, benzyl hyaluronate.

Hyaluronic acid or the pharmaceutically acceptable salt or ester thereof may be present in w/w concentration of from 0.01% to 5%, more preferably from 0.025% to 4%, most preferably from 0.04% to 2.0%.

The C₁-C₄ alkanol may be present in w/w concentration of from 0.5% to 15.0%, more preferably from 1.0% to 10.0%, most preferably from 3.0% to 7.0%. It is preferably selected from ethanol, propanol or isopropanol, ethanol being the most preferred one.

Pharmaceutical compositions, medical devices and cosmetics may be prepared according to conventional techniques, may contain acceptable excipients, adjuvants and/or carriers, and may also contain, in combination, one or more active principles with complementary or, in any case, useful activity.

The active agents which may be used in combination with the composition object of the present invention include, but are not limited to, moisturizing agents, emollients, anti-oxidants, and vitamins; the excipients which may be used include, but are not limited to humectants, rheological additives, emulsifiers, emollients, preservatives, penetration enhancers such as liposome vesicles of phosphatidylcholine; natural, synthetic and semi-synthetic polymers and co-polymers, silicone derivatives, powders and fillers, with texturizing and soft-focus effects.

Examples of the compositions in accordance to the present invention include: cream, gel, ointment, solution, emulsion, suspension for topical application.

The pharmaceutical compositions and the uses of the present invention will now be more fully described by the following examples. It should, however, be noted that such examples are given by way of illustration and not of limitation.

### EXAMPLE 1:

A formulation in gel form is prepared with the following composition in p.b.w.(%):

| | |
|---|---|
| Propylene Glycol | 12.00% |
| Denatured Ethanol | 5.00% |
| *Humulus lupulus* Hydroalcoholic Extract | 1.00% |
| Phosphatidylcholine | 0.85% |
| Carbomer* | 0.75% |
| Sodium Methylparaben | 0.26% |
| Imidazolidinil Urea | 0.20% |
| Sodium Hydroxide | 0.12% |
| Disodium EDTA | 0.10% |
| Sodium Hyaluronate | 0.05% |
| Sodium Propylparaben | 0.03% |
| Vitamin E Acetate | 0.02% |
| Cholesterol | 0.01% |
| Purified Water | q.d.s. to 100.00% |

| | |
|---|---|
| * The term "Carbomer" is intended to mean homopolymers of acrylic acid crosslinked with polyalkenyl polyether. a) the following ingredients are dissolved: Propylene Glycol 12% (p.b.w.) - Sodium Methylparaben 0.26% - Sodium Propylparaben 0.03% - Disodium EDTA 0.10% - Carbomer 0.75%, in purified water (p.b.w.) b) the following ingredients are dissolved: Vitamin E Acetate 0.02% - Phosphatidylcholine 0.85% - Cholesterol 0.01% in Ethyl Alcohol 5% (p.b.w.) c) a) and b) are heated separately at 60°C and combined, homogenizing with a suitable turbo mixer; the compound is cooled down to 40°C and Lupulus Mother Tincture (p.b.w.) 1% is added under gentle stirring; pre-mixed Sodium Hyaluronate 0.05% in part of total purified water, forms an homogeneous gel that is added to the compound under stirring. d) Imidazolidinyl Urea 0.20% and Sodium Hydroxide 0.12%, pre-mixed in part of total purified water, are added on sequence under stirring and the compound mixed thoroughly, until to obtain an homogeneous gel. | |

### COMPARATIVE EXAMPLE 2

To evaluate the synergistic activity of the composition according to the Example 1 by a biological method, the following compositions have been prepared:

### COMPARATIVE COMPOSITION 2

A formulation in gel form is prepared with the following composition in p.b.w.(%):

| | |
|---|---|
| Propylene Glycol | 12.00% |
| Denatured Ethanol | 5.00% |
| Phosphatidylcholine | 0.85% |
| Carbomer* | 0.75% |
| Sodium Methylparaben | 0.26% |
| Imidazolidinil Urea | 0.20% |
| Sodium Hydroxide | 0.12% |
| Disodium EDTA | 0.10% |
| Sodium Propylparaben | 0.03% |
| Vitamin E Acetate | 0.02% |
| Cholesterol | 0.01% |
| Purified Water | q.d.s. to 100.00% |

### COMPARATIVE COMPOSITION 3

A formulation in gel form is prepared with the following composition in p.b.w.(%):

| | |
|---|---|
| Propylene Glycol | 12.00% |
| Denatured Ethanol | 5.00% |
| *Humulus lupulus* Hydroalcoholic Extract | 1.00% |
| Phosphatidylcholine | 0.85% |
| Carbomer* | 0.75% |
| Sodium Methylparaben | 0.26% |
| Imidazolidinil Urea | 0.20% |
| Sodium Hydroxide | 0.12% |
| Disodium EDTA | 0.10% |
| Sodium Propylparaben | 0.03% |
| Vitamin E Acetate | 0.02% |
| Cholesterol | 0.01% |
| Purified Water | q.d.s. to 100.00% |

### COMPARATIVE COMPOSITION 4

A formulation in gel form is prepared with the following composition in p.b.w.(%):

| | |
|---|---|
| Propylene Glycol | 12.00% |
| Denatured Ethanol | 5.00% |
| Phosphatidylcholine | 0.85% |
| Carbomer* | 0.75% |
| Sodium Methylparaben | 0.26% |
| Imidazolidinil Urea | 0.20% |
| Sodium Hydroxide | 0.12% |
| Disodium EDTA | 0.10% |
| Sodium Hyaluronate | 0.05% |
| Sodium Propylparaben | 0.03% |
| Vitamin E Acetate | 0.02% |
| Cholesterol | 0.01% |
| Purified Water | q.d.s. to 100.00% |

### COMPARATIVE COMPOSITION 5

A formulation in gel form is prepared with the following composition in p.b.w.(%):

| | |
|---|---|
| Propylene Glycol | 12.00% |
| *Humulus lupulus* Hydroalcoholic Extract | 1.00% |
| Phosphatidylcholine | 0.85% |
| Carbomer* | 0.75% |
| Sodium Methylparaben | 0.26% |
| Imidazolidinil Urea | 0.20% |
| Sodium Hydroxide | 0.12% |
| Disodium EDTA | 0.10% |
| Sodium Hyaluronate | 0.05% |
| Sodium Propylparaben | 0.03% |
| Vitamin E Acetate | 0.02% |
| Cholesterol | 0.01% |
| Purified Water | q.d.s. to 100.00% |

Compared to the composition of the Example 1, the Comparative Composition 2 is missing both hops extract and hyaluronic acid; the Comparative Composition 3 is missing hyaluronic acid; the Comparative Composition 4 is missing hops extract, finally the Comparative Composition 5 is missing ethanol. The composition according to the Example 1 is the sole containing hops extract, hyaluronic acid and ethanol together.

### EXAMPLE 3

To the test the different antioxidant activity of the composition according to the Example 1 versus the Comparative Compositions 2-5 of the Example 2, they were tested at different concentrations (0,500-0,016 mg/ml) on Reactive oxygen species (ROS) measured on keratinocytes after exposure to Ultraviolet light A (UVA), with and without the tested sample, evaluating the cell viability after UVA stress by Neutral red uptake (NRU) test: cell survival assay using cultured human keratinocytes in monolayer cultures with and without the tested sample.

### Preparation

Human primary keratinocytes come from paediatric foreskins, with ethic committee's permission, from pre-planned routine surgery. The epidermis was separated from dermis by incubation with dispose for three and trypsinized to generate single cell suspension.

Keratinocytes were cultivated in Dulbecco's modified Eagle's and Ham's F12 media (3:1) enriched with 10% foetal calf serum (v/v) and specific enrichments.

These cells multiply in culture until a cell monolayer is reached. In this study, the cells were seeded in 96-well plates and semi-confluency (30.000 cells/well) was reached in 24 hours. Once a confluence of 60-70% has been reached, fresh medium is added with scalar dilutions of the tested sample. Non-treated cells are used as negative controls. At this stage the cell cultures were treated with different dilutions of the test compound and of the controls to obtain final concentrations ranging from 0.5 to 0.016 mg/ml. For each dilution, three replicate tests were performed. The product was dissolved in the culture medium. 0,15 mg/ml Vitamin C is added separately as positive control. Part of the cells was checked for their vitality with the NRU assay. The remaining cells were then exposed to 4' (1 J/cm2), 8' (2 J/cm2) and 12' (3 J/cm2). At the end of the exposure period, the ROS formation is investigated in the cell supernatant. The cell vitality is determined after UVA exposure and without UV exposure.

After having exposed the cells to the tested sample, the cell culture medium is removed and the cells are washed in PBS. The dichlorofluoresce in acetate (DCA) solution is added to each well. DCA is reacting with free radicals in the medium, originating a fluorescent derivative, and the fluorimeter reading allows obtaining a quantitative data related to the ROS content in the cells.

After suitable incubation, the DCA solution has been discharged and the cells have then been exposed for different times to UVA irradiation and soon after read in the fluorimeter, as described (Toxicol. Letters 1997 - 93:47-54).

The NRU assay is based on the cell ability to incorporate and bind the Neutral Red (NR), a vital dye. The NRU is a week cationic dye that penetrates the cell membrane through a mechanism of non-ionic diffusion and that is accumulated in the lysosomes, on matrix anionic sites. Cell and lysosome membrane alterations cause lysosomes fragility and gradual irreversible changes in the cells. These changes induced by xenobiotics determinate the decrease of NR uptake and of its linkage to lysosomes. This method is able to discriminate alive, damaged or dead cells. Cells are incubated with scalar concentrations of the products and with the Neutral Red solution (NR). If the membrane is damaged, it releases the dye in the medium.

After incubation, the medium is replaced with fresh medium + NR medium and cells are incubated for 4h at 37°C. Then cells are washed more times to eliminate exceeding dye wastes and read at the colorimeter.

The results are expressed in terms of viability: % cell viability = OD treated cells x 100/ OD untreated control cells.

The results, which are summarized in Table I, showed that at the sub-toxic tested concentrations (0,016 mg/ml and 0,031 mg/ml), the study product was able to reduce ROS production after UVA stress (at short time exposure).

**Table I**

| Composition | mg/ml | 4' UVA | 8'UVA | 12'UVA |
|---|---|---|---|---|
| Example 1 | 0.031, | 12.0 | 10.5 | 12.7 |
| | 0.016 | < | 14.0 | 19,4 |
| Comparative Composition 2 | 0.031 | < | < | < |
| | 0.016 | < | < | < |
| Comparative Compositions 3 | 0.031 | < | < | < |
| | 0.016 | < | 13.6 | 11.7 |
| Comparative Composition 4 | 0.031 | < | < | < |
| | 0.016 | 12.8 | 11.2 | < |
| Comparative Composition 5 | 0.031 | < | < | < |
| | 0.016 | < | < | < |
| Vitamin C | 0.15 | 19.6 | 13.4 | 14.8 |

Chosen a threshold quote of < 10% of ROS inhibition, the anti ROS effect with the testing product showed a superiority compared to the other formulations without *Humulus lupulus,* hyaluronic acid and/or ethanol, having achieved a superiority even above the control (vitamin C at 0,15 mg/ml), with a concentration 10- and 5-fold higher than the test product.

### EXAMPLE 4

To the test the different collagen regenerating activity of the composition according to the Example 1 versus the Comparative Compositions 2-5 of the Example 2, they were tested at different concentrations (2.50%, 1.25%, 0.63%), by means of in vitro evaluation of the collagen synthesis in human skin fibroblasts exposed to treatment with the tested compositions. The ex-novo synthesis of the collagen was measured by means of colorimetric assay.

### Preparation

Tested product was diluted in cell culture medium to achieve the final concentrations chosen for the tests. The product was tested at 20%, 10%, 5%, 2.50%, 1.25%, 0.63 and 0.31% (w/v) for preliminary cytotoxicity test. In accordance with toxicity data (IC₅₀= 6.79%, NON CYTOTOXIC), 3 different non-cytotoxic concentrations were chosen to continue the tests. The concentrations chosen for the efficacy test are 2.50%, 1.25% and 0.63% (w/v). Cell exposure to test product was prolonged for 24 and 48 hours. At end of each experimental time, neo-synthesis of extracellular matrix element was measured.

The determination of collagen synthesis is carried out by quantitative dye-binding method. The chromogen agent used in the assay is Sirius Red (Direct red 80). These groups react with the side chain groups of the basic amino acids of collagen. The specific affinity of the dye for collagen, under the assay conditions, is due to the elongated dye molecules becoming aligned parallel to the long, rigid structure of native collagen that have intact triple helix organisation (dye affinity is much reduced when collagen is denatured). Collagen concentration (µg in 20 µl of medium) is calculated by means of data interpolation on a standard curve obtained with known and increasing collagen concentrations. The product test at all tested concentrations resulted significantly effective in terms of speed in increasing collagen synthesis compared to non-treated cells at all of the monitored experimental times (the variation between collagen content in the cells treated with the study product at 0.63% for 48 hours is not significant compared to the collagen content in the untreated cell culture) and to the other formulations without *Humulus lupulus*, hyaluronic acid and/or ethanol. The increase has a dose-dependent trend. The results are summarized in Table II.

**Table II**

| | | Collagen Synthesis % | |
|---|---|---|---|
| Composition | mg/ml | 24h | 48h |
| Example 1 | 2.5% | 56.2% | 34.4% |
| | 1.25% | 76.7% | 29.0% |
| | 0.63% | 43.6% | 7.7% |
| Comparative Composition 2 | 2.5% | 20.7% | 48.9% |
| | 1.25% | 39.7% | 20.0% |
| | 0.63% | 1.8% | 12.3% |
| Comparative Composition 3 | 2.5% | 17.1% | 62.5% |
| | 1.25% | 21.3% | 38.5% |
| | 0.63% | 0.8% | 23.3% |
| Comparative Composition 4 | 2.5% | 45.7% | 22.2% |
| | 1.25% | 71.2% | 15.2% |
| | 0.63% | 48.3% | 11.8% |
| Comparative Composition 5 | 2.5% | 13.9% | 21.1% |
| | 1.25% | 9.2% | 7.0% |
| | 0.63% | 8.1% | -1.4% |

## Claims

1. A composition containing:
a)an extract of *Humulus lupulus,*
b)hyaluronic acid or a pharmaceutically acceptable salt or ester thereof,
c) a C₁-C₄ alkanol.

2. The composition according to claim 1, **characterized in that** said extract is a liquid, semi-solid or solid extract.

3. The composition according to claim 1, **characterized in that** said liquid extract is a mother tincture.

4. The composition according to claim 1, **characterized in that** the amount of said extract is from 0.1 to 15% by weight of the total composition, preferably from 0.2 to 5%.

5. The composition according to claim 4, **characterized in that** said amount is from 0.5 to 2.5%.

6. The composition according to claim 1, **characterized in that** said pharmaceutically acceptable salt is selected from sodium salt, potassium salt, calcium salt or a salt with a natural aminoacid.

7. The composition according to claim 1, **characterized in that** said pharmaceutically acceptable ester is selected from ascorbyl hyaluronate, palmitoil hyaluronate, benzyl hyaluronate.

8. The composition according to claim 1, **characterized in that** the amount of hyaluronic acid or a pharmaceutically acceptable salt thereof is from 0.01 to 5% by weight of the total composition, preferably from 0.025 to 4%.

9. The composition according to claim 8, **characterized in that** said amount is from 0.04 to 2.0%.

10. The composition according to claim 1, **characterized in that** the C₁-C₄ alkanol is ethanol.

11. The composition according to claim 1, **characterized in that** amount of the C₁-C₄ alkanol is from 0.5 to 15% by weight of the total composition, preferably from 1 to 10%.

12. The composition according to claim 11, **characterized in that** said amount is from 3 to 7%.

13. The composition according to anyone of the preceding claims, **characterized by** further containing one or more of the following: moisturizing agents, emollients, anti-oxidants, penetration enhancers such as liposome vesicles and vitamins; humectants, rheological additives, emulsifiers, emollients, preservatives; natural, synthetic or semi-synthetic polymers and co-polymers, silicone derivatives, powders and fillers, with texturizing and soft-focus effects.

14. A composition according to any one of the preceding claims, for use in the treatment and prevention of skin ageing and/or wrinkles.

15. The composition for use according to claim 14, **characterized in that** the recipient of such a treatment is a human.
